# EUROPEAN PATENT APPLICATION

(11) **EP 4 635 485 A1**
(43) Date of publication of application: **22.10.2025**
(21) Application number: 24909220.6
(22) Date of filing: 19.01.2024
(51) Int. Cl.: A61K 9/36

(54) **NICOTINE MICROPELLET COMPOSITION, NICOTINE MICROPELLET ORAL POUCH, AND METHOD FOR PREPARING SAME**

(71) Applicant: Maifeng (Zhuhai) Technology Co., Ltd., Zhuhai, Guangdong 519000 (CN)
(72) Inventor: LIN, Shaohui, Zhuhai, Guangdong 519000 (CN); HU, Yongwei, Zhuhai, Guangdong 519000 (CN)
(74) Representative: Chung, Hoi Kan
(86) International application number: PCT/CN2024/073251
(87) International publication number: WO 2025/152148

(57) **Abstract**

The present invention relates to a nicotine pellet composition, a nicotine pouch and a preparation method thereof. The nicotine pellet composition comprises nicotine, a blank pellet core, a binding agent, a sweetener, a pH adjuster, cellulose and cellulose derivatives, a polymer coating material, a lubricant, flavors and fragrances and a solvent, the blank pellet core is loaded with the nicotine, and the polymer coating material forms a single layer or multiple layers of drug carrier or coating on the blank pellet core. When the polymer coating material forms multiple layers of drug carrier or coating, it includes an isolation layer, a drug-containing layer, a shielding layer and a drug carrying layer from inside out. The present invention provides a nicotine pellet composition and a nicotine pouch with good fluidity, stable nicotine release and stable aroma release, which can be used for the treatment of smoking cessation or nicotine dependence.

## Description

### Technical field

The present invention relates to the technical field of nicotine compositions, especially a nicotine pellet composition, nicotine pouch and preparation method thereof.

### Background technology

Pouches of tobacco free oral nicotine products are gaining rising popularity due to perceived harm reduction, ease of consumption and novel taste thereof. With the products, users can enjoy nicotine without tobacco, for oral nicotine products such as nicotine pouches, usually the nicotine pouches are placed between lips and gums for some time, during which nicotine is absorbed directly through the oral mucosa, free from harm due to nitrosoamine, smoke or gas and a plurality of carcinogenic substances associated with conventional tobacco products, nicotine pouches are generally used for quitting tobacco or treatment for nicotine dependence.

For existing nicotine pouches, usually nicotine and accessories are mixed to be pellets and packed in non-woven pouches. However, nicotine particles may agglomerate and are prone to adhere to the surfaces of containers or packing materials, especially where sizes of the nicotine particles are reduced, tendency of particle agglomeration and agglomeration strength is increased, meanwhile, as shapes and sizes of the particles are different, releasing rates of nicotine are not stable. Furthermore, fluidity of the nicotine particles is subjected to influences of flavors and moistures and may become poor, in addition, nicotine decomposition will dissociate nicotine alkali, and free nicotine alkali will outperform scents of flavorings and unpleasant smells may appear, meanwhile, product stability is poor, shelf life thereof is reduced.

Chinese patent publication no. CN107259634A published on 20 October 2017 discloses an oral product, comprising a main body that can be consumed in an oral cavity, wherein the main body comprises: an extruded orally stable polymer substrate, wherein an oral product comprises one or more orally stable polymers having a weight by percentage 10%, selected from polyurethane, polyacrylate, polyethylene, SEBS, SBS or any combination thereof; cellulose base fiber embedded in the orally stable polymer substrates having a weight by percentage at least 10%, forming jointly a cellulose-polymer substrate; and nicotine or a derivative thereof dispersed or absorbed in the cellulose-polymer substrate so as to release the nicotine or the derivative thereof from the main body where the main body is consumed in the oral cavity and contacts saliva, wherein the main body has a compressive coefficient in between 45% and 90% under 250 N, and elasticity percentage in between 75% and 90%. In this patent application, extrusion and shaping processes are used to make the nicotine composition, comprising nicotine compositions having spherical shapes. This patent application has not disclosed empty pellet cores and nicotine pellet compositions made from nicotine and accessories.

Chinese patent application publication no. CN114521669A published on 24 May 2022 disclosed an oral tobacco product containing stable nicotine, comprises binding agents, nicotine, pH adjusting agents, core materials and fillers. Preparation method thereof comprises: (1) preparation of nicotine stable mixed solution: dissolving high polymers in absolute ethyl alcohol, mixing evenly and adding the nicotine and the pH adjusting agent; (2) mixing the fillers and the solution obtained in step (1), and mixing the solution with sweeteners, flavorings, moisturizers evenly; and (3) adding core materials in the solution obtained in the step (2), stirring for absorption, drying overnight, and obtaining flavored nicotine stable oral tobacco particles. Apparently, with this preparation method, it is next to impossible to guarantee the nicotine contents in each of the pellets are equal.

In the prior art, no nicotine product having good fluidity, stable nicotine release and stable flavor release is available.

Therefore, technical improvements are demanded.

### Summary of invention

In the prior art, no nicotine product of high fluidity and being able to release nicotine and scents stably is available, therefore, the present invention provides a nicotine pellet composition, a nicotine pouch and a preparation method for solving the listed problems.

To realize the foregoing purpose, on one aspect, the present invention provides a nicotine pellet composition, comprising nicotine, a blank pellet core, a binding agent, a sweetener, a pH adjusting agent, a cellulose and cellulose derivative, a high polymer coating material, a lubricant, a flavoring, and a solvent, wherein the blank pellet core carries the nicotine, and the high polymer coating material forms a single-layered or multi-layered carrier or coating.

In an implementation method, wherein the high polymer coating material forms the multi-layered carrier or coating, the multi-layered carrier or coating comprise from inside out an isolating layer, a drug containing layer, a shielding layer and a drug carrying layer.

In an implementation method, the nicotine comprises any one or a combination of free base nicotine, nicotine salts, nicotine in a matrix or organometallic complex, nicotine-ion exchange resin combinations, nicotine inclusion complexes or any non-covalently bound nicotine, nicotine lactate, nicotine malate, nicotine salicylate, nicotine resin complexes, nicotine cyclodextrin inclusion complexes, nicotine hydrochloride, nicotine dihydrochloride, nicotine tartrate, nicotine bitartrate dihydrate, nicotine sulfate, nicotine zinc chloride and nicotine benzoate.

In an implementation method, the blank pellet core comprises hydrophilous glycosyl based cores and /or hydrophobic cellulose cores, wherein the hydrophilous glycosyl based cores comprise pellet cores made from saccharide materials soluble in water, and the hydrophobic cellulose cores comprise pellet cores made from cellulose materials insoluble in water.

In an implementation method, the binding agent comprises any one or a combination of polyvidone, cellulose derivatives, amylum pregelatinisatum, gelatin, gum arabic, sodium alginate and polyethylene glycol.

In an implementation method, the sweetener comprises any one or a combination of acesulfame potassium, sodium cyclamate, saccharin, sodium saccharin, sucralose, neotame, aspartame, stevia, licorice, disodium glycyrrhizinate, tripotassium glycyrrhizinate and trisodium glycyrrhizinate, glucose, fructose, sucrose, maltose, starch-based sugar and lactose, sorbitol, naltitol, isomalt, xylitol, lactitol, mannitol and erythritol.

In an implementation method, the pH adjusting agent comprises any one or a combination of acetates, alkali metal or ammonium carbonates, lactates, glycinates, gluconates, borates, glycerophosphates or citrates, phosphates and metal hydroxides.

In an implementation method, the cellulose or cellulose derivative comprises any one or a combination of wheat fiber, pea fiber, rice fiber, corn fiber, oat fiber, tomato fiber, barley fiber, rye fiber, beet fiber, buckwheat fiber, potato fiber, cellulose fiber, apple fiber, cocoa fiber, bran fiber, bamboo fiber, pandan fiber, powdered cellulose and high molecular polymer materials.

In an implementation method, the high polymer coating material comprises glycosyl based materials or high polymers, wherein the glycosyl based materials comprise any one or a combination of syrups, colored syrups, mucilages, talcum powders and cera chinensis; and the high polymers comprise any one or a combination of hydroxypropyl methylcellulose, hydroxypropyl cellulose, hydroxyethyl cellulose, methyl cellulose, methacrylic acid copolymers, methacrylate copolymers, cellulose acetate phthalate, polyvinyl pyrrolidone, polyvinyl pyrrolidine and polyethylene glycol.

In an implementation method, the lubricant comprise hydrophobic lubricants and hydrophilous lubricants, wherein the hydrophobic lubricants comprise any one or a combination of stearic acid, magnesium stearate, calcium stearate, glyceryl behenate, micronized silica, talc, vegetable oil, polyethylene glycol and sodium lauryl sulfate and the hydrophilous lubricants comprise sodium stearyl fumarate.

In an implementation method, the flavoring comprises any one or a combination of bergamot flavor, eucalyptus flavor, citrus flavor, lemon flavor, peppermint flavor, mint flavor, menthol, licorice flavor, wintergreen flavor, tobacco flavor, coffee flavor, vanilla flavor, lime flavor, apple flavor, peach flavor, mango flavor, cherry flavor, blueberry flavor, strawberry flavor, cola flavor, cinnamon flavor and watermelon flavor.

In an implementation method, the solvent comprises water and/or organic solvents, wherein the organic solvents comprise any one or a combination of aromatic hydrocarbons, aliphatic hydrocarbons, alicyclic hydrocarbons, halogenated hydrocarbons, alcohols, ethers, esters, ketones, diol derivatives, others such as acetonitrile, pyridine and phenol.

On another aspect, the present invention further provides a nicotine pouch, comprising a non-woven pouch and the nicotine pellet composition as set forth in the foregoing paragraphs in the non-woven pouch.

On the third aspect, the present invention further provides a preparation method of the nicotine pouch, for preparing the nicotine pouch, comprising:
S1: weighing and proportioning: weighing materials; proportioning the high polymer coating material according to a formulation;
S2: drug carrying and coating: after proportioning of the high polymer coating material, placing into a material silo having a sprayer, placing weighed solid materials sequentially into another material silo having a fluidized bed or a centrifugal coating machine, starting coating using the fluidized bed or the centrifugal coating machine, coating the solid materials evenly over the blank pellet core to form the single-layered or multi-layered coating and obtaining nicotine pellets;
S3: drying: placing the nicotine pellets into a drying box or the fluidized bed for drying; and
S4: packaging: transferring the nicotine pellets after drying to a powder silo of a granule packaging machine, placing rolls of non-woven cloths into a film silo of the granule packaging machine, starting the granule packaging machine for packaging, so to enclose the nicotine pellets into non-woven parcels and obtaining the nicotine pouch.

Beneficial effects: in the present invention, by forming the single-layered or multi-layered drug-carriers or coatings over the blank pellet cores, the nicotine pellet composition and the nicotine pouch can release nicotine quickly, uniformly and stably, contents and release of nicotine are stable, in the meanwhile, scent release is stable too; by forming the drug-carrying layer or the coating layer, moisture proof, sunlight shielding and air isolation can be realized, and stability of the nicotine pellet composition is improved; with the preparation method provided in the present invention, glidants are not required for packaging, a weight difference thereof is lower than that for powder and particle filling and packaging, mutual interactions between ingredients during preparation are avoided, so that the fluidity of the nicotine pellet composition and the nicotine pouch is good. The present invention provides a nicotine product having good fluidity, releasing nicotine and flavors stably, which can be used for tobacco addiction or dependence treatment.

### Brief description of drawings

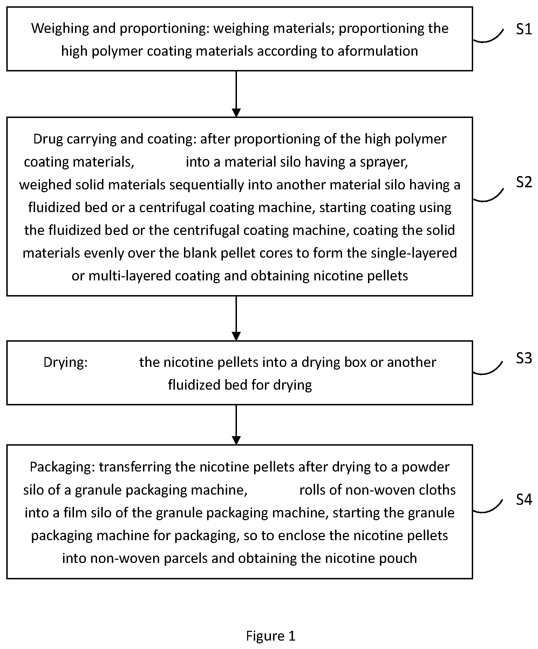

Figure 1 is a flow chart diagram showing a preparation method of a nicotine pouch provided in the present invention.

Implementation of the technical solutions of the present invention, functions, features and advantages thereof will be further given in conjunction with the embodiments and with reference to the drawings.

### Embodiments

In order to make the purpose, technical solutions and advantages of the present invention more clearly understood, the present invention is further described in detail below in conjunction with the accompanying drawings and embodiments. It should be understood that the specific embodiments described herein are only used to explain the present invention, and are not used to limit the present invention. In addition, the terms "one embodiment", "some embodiments", "example", "specific example", or "some examples" described below mean that the specific features, structures, materials or characteristics described in conjunction with the embodiment or example are included in at least one embodiment or example of the present invention. In the present specification, the exemplary expressions of the above terms do not necessarily refer to the same embodiment or example. Moreover, the technical features involved in embodiments of the present invention can be combined with each other as long as there is no conflict between them. The present invention provides a nicotine pellet composition, comprising nicotine, blank pellet cores, binding agents, sweeteners, pH adjusting agents, cellulose and cellulose derivatives, high polymer coating materials, lubricants, flavorings and solvents, wherein the blank pellet cores carry the nicotine, and the high polymer materials form a single-layered or multi-layered drug carrier or coating over the blank pellet cores. Where the high polymer materials form the multi-layered drug carrier or coating, the multi-layered drug carrier or coating comprises from inside out sequentially an isolation layer, a drug containing layer, a shielding layer and a drug carrying layer. Wherein, where non-functional coating materials are used on the blank pellet cores for single-layered coating, during use, the nicotine can release quickly, and where functional coating materials are used on the blank pellet cores for multi-layered coating, during use the nicotine can be released evenly and slowly.

Wherein the nicotine comprises any one or a combination of free base nicotine, nicotine salts, nicotine in a matrix or organometallic complex, nicotine-ion exchange resin combinations, nicotine inclusion complexes or any non-covalently bound nicotine, nicotine lactate, nicotine malate, nicotine salicylate, nicotine resin complexes, nicotine cyclodextrin inclusion complexes, nicotine hydrochloride, nicotine dihydrochloride, nicotine tartrate, nicotine bitartrate dihydrate, nicotine sulfate, nicotine zinc chloride and nicotine benzoate. Wherein the nicotine comprises synthesized nicotine and nicotine extracted from natural tobacco.

Wherein the blank pellet cores comprise masterbatches required for preparing and producing matrix pellets. Wherein the blank pellet cores comprise hydrophilous glycosyl based cores and /or hydrophobic cellulose cores being spherical or close to spherical and having diameters less than 25 mm. Wherein the hydrophilous glycosyl based cores comprise pellet cores made from saccharide materials soluble in water, and the hydrophobic cellulose cores comprise pellet cores made from cellulose materials insoluble in water. Further the hydrophilous glycosyl based cores comprise sucrose blank pellet cores or starch blank pellet cores, wherein the hydrophobic cellulose cores comprise microcrystalline cellulose blank pellet cores.

Wherein the binding agents comprise pharmaceutically acceptable binding agents. Wherein the binding agents comprise any one or a combination of polyvidone, cellulose derivatives, amylum pregelatinisatum, gelatin, gum arabic, sodium alginate and polyethylene glycol.

Wherein the sweeteners comprise any one or a combination of acesulfame potassium, sodium cyclamate, saccharin, sodium saccharin, sucralose, neotame, aspartame, stevia, licorice, disodium glycyrrhizinate, tripotassium glycyrrhizinate and trisodium glycyrrhizinate, glucose, fructose, sucrose, maltose, starch-based sugar and lactose, sorbitol, naltitol, isomalt, xylitol, lactitol, mannitol and erythritol.

Wherein the pH adjusting agents comprise any one or a combination of acetates, alkali metal or ammonium carbonates, lactates, glycinates, gluconates, borates, glycerophosphates or citrates, phosphates and metal hydroxides.

Wherein the cellulose and cellulose derivatives comprise any one or a combination of wheat fiber, pea fiber, rice fiber, corn fiber, oat fiber, tomato fiber, barley fiber, rye fiber, beet fiber, buckwheat fiber, potato fiber, cellulose fiber, apple fiber, cocoa fiber, bran fiber, bamboo fiber, pandan fiber, powdered cellulose and high molecular polymer materials. Wherein the cellulose and cellulose derivatives and/or high polymer materials are permeable to saliva, and partially or completely dissolved in the nicotine pellet composition.

Wherein the high polymer coating materials comprise glycosyl based materials or high polymers, wherein the glycosyl based materials comprise any one or a combination of syrups, colored syrups, mucilages, talcum powders and cera chinensis; and the high polymers comprise any one or a combination of hydroxypropyl methylcellulose, hydroxypropyl cellulose, hydroxyethyl cellulose, methyl cellulose, methacrylic acid copolymers, methacrylate copolymers, cellulose acetate phthalate, polyvinyl pyrrolidone, polyvinyl pyrrolidine and polyethylene glycol. Wherein the high polymer coating materials are coated on the solids and become one or more layers of multifunctional protective layers having different thicknesses or flexibilities, and are soluble in saliva.

Wherein the lubricants comprise hydrophobic lubricants and hydrophilous lubricants, wherein the hydrophobic lubricants comprise any one or a combination of stearic acid, magnesium stearate, calcium stearate, glyceryl behenate, micronized silica, talc, vegetable oil, polyethylene glycol and sodium lauryl sulfate and the hydrophilous lubricants comprise sodium stearyl fumarate. And the lubricants are configured to reduce mutual friction in between particles or powders to enhance fluidity, lubrication and resistance to bond.

Wherein the flavorings comprise any one or a combination of bergamot flavor, eucalyptus flavor, citrus flavor, lemon flavor, peppermint flavor, mint flavor, menthol, licorice flavor, wintergreen flavor, tobacco flavor, coffee flavor, vanilla flavor, lime flavor, apple flavor, peach flavor, mango flavor, cherry flavor, blueberry flavor, strawberry flavor, cola flavor, cinnamon flavor and watermelon flavor.

Wherein the solvents comprise water and/or organic solvents, wherein the organic solvents comprise any one or a combination of aromatic hydrocarbons, aliphatic hydrocarbons, alicyclic hydrocarbons, halogenated hydrocarbons, alcohols, ethers, esters, ketones, diol derivatives, others such as acetonitrile, pyridine and phenol.

On another aspect, the present invention further provides a nicotine pouch, comprising a non-woven pouch and the nicotine pellet composition as set forth in the foregoing paragraphs in the non-woven pouch.

On the third aspect, the present invention further provides a preparation method of the nicotine pouch, for preparing the nicotine pouch, comprising:
S1: weighing and proportioning: weighing materials; proportioning the high polymer coating materials according to the formulation;
S2: drug carrying and coating: after proportioning of the high polymer coating materials, placing into a material silo having a sprayer, placing weighed solid materials sequentially into another material silo having a fluidized bed or a centrifugal coating machine, starting coating using the fluidized bed or the centrifugal coating machine, coating the solid materials evenly over the blank pellet cores to form the single-layered or multi-layered coating and obtaining nicotine pellets;
S3: drying: placing the nicotine pellets into a drying box or another fluidized bed for drying; and
S4: packaging: transferring the nicotine pellets after drying to a powder silo of a granule packaging machine, placing rolls of non-woven cloths into a film silo of the granule packaging machine, starting the granule packaging machine for packaging, so to enclose the nicotine pellets into non-woven parcels and obtaining the nicotine pouch.

The present invention provides the following embodiments to explain the technical solutions of the present invention.

Embodiment 1 preparing nicotine pouch capable of quick nicotine release, a preparation process thereof, and in vitro dissolution profile.

Materials required were weighed as per the formulation as set forth in table 1.

**Table 1 proportioning of nicotine pellet composition in embodiment 1:**

| S. N. | Description | Theoretical quantity (g) | Actually weighed quantity (g |
|---|---|---|---|
| 1 | Sucrose pellets | 500.00 | 500.00 |
| 2 | 95% ethanol | 100.00 | 100.00 |
| 3 | Nicotine bitartrate dihydrate | 18.50 | 18.50 |
| 4 | Neotame | 0.33 | 0.33 |
| 5 | Cooling agent | 0.65 | 0.65 |
| 6 | Polyvidone | 13.00 | 13.00 |
| 7 | Flavorings | 32.50 | 32.50 |

Wherein materials in table 1 are prepared as per the following method:
Step 1: preparing drug containing coating liquid: taking 95% ethanol 100 g, heating until 50 °C, pouring in nicotine bitartrate dehydrate, shearing and dispersing for 5 minutes using an emulsion machine until the dispersion is clear. Cooling down until 30 °C, adding polyvidone, shearing and dispersing for 5 minutes using the emulsion machine until complete dissolution. Adding in sequence neotame, shearing and dispersing for 5 minutes until complete dissolution using the emulsion machine. Adding the cooling agent, shearing and dispersing for 5 minutes using the emulsion machine until complete dissolution. Finally adding the flavorings, and shearing and dispersing for 10-15 minutes until complete dissolution.
Step 2: drug carrying and coating: adding the blank sucrose pellets to a silo of the centrifuge pelletizing and coating machine, putting the drug containing coating liquid prepared in step 1 into a sprayer silo, setting parameters of the machines as per table 2, starting the machines for centrifuge coating for 6 minutes until the drug containing coating liquid is coated over the sucrose pellets and forms a drug containing layer.

**Table 2 parameters for preparing processes in embodiment 1**

| Air inlet temperature °C | Material temperature °C | Air inlet pressure kpa | Revolution rate r/min | Spraying revolution rate r/min | Atomization pressure bar |
|---|---|---|---|---|---|
| 45-55 | 41-46 | 1.50 | 60.0 | 5-10 | 1.0 |

Step 3: drying: putting the pellets obtained in the step 2 in a drying oven, drying at a temperature of 45 °C for 4-6 hours.
Step 4: packing: transferring the dried nicotine pellets obtained in step 3 to a powder silo of the pellet packaging machine, putting non-woven rolls into a film silo of the pellet packaging machine; and starting the pellet packaging machine.

In vitro dissolution experiments were given to the nicotine pouches obtained in the embodiment 1 and the experiment data are shown in table 3.

**Table 3 in vitro dissolution profile of the nicotine pellets capable of quick nicotine release obtained in the embodiment 1**

| Time | Dissolution quantity |
|---|---|
| 5 minutes | 65% |
| 10 minutes | 87% |
| 15 minutes | 96% |
| 20 minutes | 100% |

From the foregoing embodiment 1, it can be known that, the nicotine pellets and nicotine pouches prepared by pellet coating as per the centrifugal pelletizing and coating process can release nicotine quickly.

Embodiment 2 Preparing nicotine pellet pouches releasing nicotine slowly, preparation process thereof, and in vitro dissolution profile.

The materials required were weighed as per the proportions listed in table 4.

**Table 4:**

| S. N. | Description | Percentage % |
|---|---|---|
| Isolation layer | | |
| 1 | Sucrose pellets | 90.0 |
| 2 | Ethylcellulose | 7.1 |
| 3 | Magnesium stearate | 2.9 |
| 4 | Ethanol | - |
| Sub total | | 100 |

| Drug containing layer | | |
|---|---|---|
| 6 | Sucrose pellets after being coated with the isolation layer | 80.0 |
| 7 | Nicotine bitartrate dihydrate | 6.0 |
| 8 | Polyvidone | 8.8 |
| 9 | Acesulfame potassium | 2.0 |
| 10 | Magnesium Stearate | 1.0 |
| 11 | Flavoring | 1.2 |
| 12 | Sodium carbonate aqueous solution | 1.0 |
| 13 | Ethanol | - |
| Sub total | | 100 |

| Shielding layer coating | | |
|---|---|---|
| 14 | Nicotine pellets after being coated with the drug containing coating | 95.0 |
| 15 | Polyvidone | 3.4 |
| 16 | Magnesium stearate | 1.6 |
| 17 | Ethanol | - |
| Sub total | | 100 |

| Drug-carrying layer coating | | |
|---|---|---|
| 18 | Nicotine pellet after being coated with the shielding layer | 63.5 |
| 19 | Methacrylic acid-ethyl acrylate copolymer | 25.8 |
| 20 | Sodium carbonate aqueous solution | 1.2 |
| 21 | Nicotine bitartrate dihydrate | 3.0 |
| 22 | Neotame | 0.1 |
| 23 | Flavorings | 6.4 |
| 24 | Purified water | - |
| 25 | Ethanol | - |
| Sub total | | 100.0 |

Wherein the materials listed in table 4 were prepared as per the following method:

### A: isolation layer coating process

Step A1: preparing the isolation layer coating liquid: dissolving the ethylcellulose and the magnesium stearate of the specified quantity using 95% ethanol. Weighing blank sucrose pellets of the specified quantity.

Step A2: isolation layer coating: putting the prepared ethylcellulose and magnesium stearate solution into the sprayer silo, putting the weighed blank sucrose pellets into a fluidized bed solid silo, setting the parameters of the fluidized bed as per table 5, starting the machine for fluidized bed coating, in the meanwhile, drying using the fluidized bed for 60 minutes, and coating uniformly the ethylcellulose and magnesium stearate solution over the sucrose pellets to form the isolation layer.

**Table 5 parameters for the isolation layer coating process**

| Air inlet temperature °C | Material temperature °C | Upper/lower atomization pressure bar | Air inlet quantity m³/h | Humidity g/kg |
|---|---|---|---|---|
| 40-65 | 41-60 | 0.5-2.2/0.5-2.2 | 1500-2500 | 3.8-7.6 |

Step A3: drying: coating the isolation layer and completing pellet drying

### B. drug-containing layer coating

Step B1: preparing a drug-containing coating solution: dissolving the nicotine bitartrate dehydrate, polyvidone, acesulfame potassium, magnesium stearate, flavoring and sodium carbonate aqueous solution of the specified quantity using 95% ethanol, and obtaining a solution A. weighing the sucrose pellets after being coated with isolation layers as per the specified amount.

Step B2 drug-containing layer coating: putting the solution A into the sprayer silo, putting the weighed sucrose pellets after being coated with the isolation layers into the fluidized bed solid silo, setting parameters for the fluidized bed as per table 6, starting the machine for fluidized bed coating, in the meanwhile, drying using the fluidized bed for 45 minutes, and coating uniformly the nicotine containing composition in the sucrose pellets being coated with the isolation layers.

**Table 6 parameters for drug-containing coating**

| Air inlet temperature °C | Material temperature °C | Upper/lower atomization pressure bar | Air inlet quantity m³/h | Humidity g/kg |
|---|---|---|---|---|
| 40-60 | 45-55 | 0.5-2.2/0.5-2.2 | 1500-2500 | 3.8-7.6 |

Step B3: drying: coating the drug-containing layer while drying the pellets.

### C. Shielding layer coating

Step C1: preparing shielding layer coating solution: dissolving the polyvidone and the magnesium stearate of the specified amounts using the 95% ethanol. Weighing the nicotine pellets being coated with the drug containing layer as per the specified amount.

Step C2: shielding layer coating: putting the prepared polyvidone and magnesium stearate into the sprayer silo, putting the nicotine pellets being coated with the drug containing coating layers into the fluidized bed solid silo, setting the machine parameters as per parameters in table 7, starting the machine for fluidized bed coating, in the meanwhile, drying using the fluidized bed for 30 minutes, and coating the shielding layer solution evenly over the nicotine pellets being coated with the drug-containing coatings.

**Table 7 parameters for shielding layer coating**

| Air inlet temperature °C | Material temperature °C | Upper/lower atomization pressure bar | Air inlet quantity m³/h | Humidity g/kg |
|---|---|---|---|---|
| 40-65 | 45-55 | 0.5-2.2/0.5-2.2 | 1500-2500 | 3.8-7.6 |

Step C3: drying: coating the shielding layer and drying

### D. drug-carrying layer coating

Step D1: preparing drug carrying layer coating solution: dissolving the nicotine bitartrate dehydrate, methacrylic acid-ethyl acrylate copolymer, sodium carbonate aqueous solution, neotame and flavorings of the specified quantity using 95% ethanol and obtaining a solution B. weighing the nicotine pellets being coated with the shielding layers as per the specified amount.

Step D2: drug carrying layer coating: putting the prepared solution B into the sprayer silo, putting the nicotine pellets being coated with the shielding layers into the fluidized bed solid silo, setting the machine parameters as per table 8, starting the machine for fluidized bed coating, in the meanwhile, drying using the fluidized bed for 60 minutes and coating the nicotine containing composition evenly over the sucrose pellets being coated with the isolation layer.

**Table 8 parameters for drug carrying layer coating**

| Air inlet temperature °C | Material temperature °C | Upper/lower atomization pressure bar | Air inlet quantity m³/h | Humidity g/kg |
|---|---|---|---|---|
| 40-60 | 45-55 | 0.5-2.2/0.5-2.2 | 1500-2500 | 3.8-7.6 |

Step D3: drying: coating the drug carrying layer and drying the nicotine pellets in the meanwhile. Step D4: packaging: putting the dried nicotine pellets to the powder silo of the pellet packaging machine, and putting the non-woven rolls into the film silo of the pellet packaging machine. Starting the pellet packaging machine for packaging.

Conducting in vitro dissolution check for the nicotine pellets obtained in embodiment 2, and the experiments data are shown in table 9.

**Table 9 in vitro dissolution data of the nicotine pellets releasing nicotine gently**

| Time | Dissolution |
|---|---|
| 5 minutes | 38% |
| 10 minutes | 45% |
| 15 minutes | 50% |
| 20 minutes | 56% |
| 25 minutes | 62% |
| 30 minutes | 68% |
| 35 minutes | 74% |
| 40 minutes | 79% |
| 45 minutes | 85% |
| 50 minutes | 90% |
| 60 minutes | 96% |
| 70 minutes | 100% |

From the embodiment 2, it can be known that using the fluidized bed spraying coating and drying process, the nicotine pellets and pouches made by coating the pellets can release the nicotine gently.

In the present invention, by forming the single-layered or multi-layered drug-carriers or coatings over the blank pellet cores, the nicotine pellet composition and the nicotine pouch can release nicotine quickly, uniformly and stably, contents and release of nicotine are stable, in the meanwhile, scent release is stable too; in the meanwhile, by making the nicotine and flavorings to be pellets, quality and contents of each of the pellets are nearly the same, and pharmarceutical releasing status is the same, release of the nicotine and the flavorings is synchronous until complete release; by forming the drug-carrying layer or the coating layer, moisture proof, sunlight shielding and air isolation can be realized, and stability of the nicotine pellet composition is improved; with the preparation method provided in the present invention, glidants are not required for packaging, a weight difference thereof is lower than that for powder and particle filling and packaging, mutual interactions between ingredients during preparation are avoided, so that the fluidity of the nicotine pellet composition and the nicotine pouch is good. The present invention provides a nicotine product having good fluidity, releasing nicotine and flavors stably, which can be used for tobacco addiction or dependence treatment.

The foregoing are some preferred embodiments of the present invention, and are not intended to limit the patent scope of the present invention, all equivalent structural or process modifications made based on the contents in the description and the drawings or direct or indirect use of the present invention in other correlated fields shall be covered in the protection scope of the present invention similarly.

## Claims

1. A nicotine pellet composition, comprising nicotine, a blank pellet core, a binding agent, a sweetener, a pH adjusting agent, a cellulose and cellulose derivative, a high polymer coating material, a lubricant, a flavoring, and a solvent, wherein the blank pellet core carries the nicotine, and the high polymer coating material forms a single-layered or multi-layered carrier or coating.

2. The nicotine pellet composition according to claim 1, wherein the high polymer coating material forms the multi-layered carrier or coating, the multi-layered carrier or coating comprise from inside out an isolating layer, a drug containing layer, a shielding layer and a drug carrying layer.

3. The nicotine pellet composition according to claim 1, wherein the nicotine comprises any one or a combination of free base nicotine, nicotine salts, nicotine in a matrix or organometallic complex, nicotine-ion exchange resin combinations, nicotine inclusion complexes or any non-covalently bound nicotine, nicotine lactate, nicotine malate, nicotine salicylate, nicotine resin complexes, nicotine cyclodextrin inclusion complexes, nicotine hydrochloride, nicotine dihydrochloride, nicotine tartrate, nicotine bitartrate dihydrate, nicotine sulfate, nicotine zinc chloride and nicotine benzoate.

4. The nicotine pellet composition according to claim 1, wherein the blank pellet core comprises hydrophilous glycosyl based cores and /or hydrophobic cellulose cores, wherein the hydrophilous glycosyl based cores comprise pellet cores made from saccharide materials soluble in water, and the hydrophobic cellulose cores comprise pellet cores made from cellulose materials insoluble in water.

5. The nicotine pellet composition according to claim 1, wherein the binding agent comprises any one or a combination of polyvidone, cellulose derivatives, amylum pregelatinisatum, gelatin, gum arabic, sodium alginate and polyethylene glycol.

6. The nicotine pellet composition according to claim 1, wherein the sweetener comprises any one or a combination of acesulfame potassium, sodium cyclamate, saccharin, sodium saccharin, sucralose, neotame, aspartame, stevia, licorice, disodium glycyrrhizinate, tripotassium glycyrrhizinate and trisodium glycyrrhizinate, glucose, fructose, sucrose, maltose, starch-based sugar and lactose, sorbitol, naltitol, isomalt, xylitol, lactitol, mannitol and erythritol.

7. The nicotine pellet composition according to claim 1, wherein the pH adjusting agent comprises any one or a combination of acetates, alkali metal or ammonium carbonates, lactates, glycinates, gluconates, borates, glycerophosphates or citrates, phosphates and metal hydroxides.

8. The nicotine pellet composition according to claim 1, wherein the cellulose or cellulose derivative comprises any one or a combination of wheat fiber, pea fiber, rice fiber, corn fiber, oat fiber, tomato fiber, barley fiber, rye fiber, beet fiber, buckwheat fiber, potato fiber, cellulose fiber, apple fiber, cocoa fiber, bran fiber, bamboo fiber, pandan fiber, powdered cellulose and high molecular polymer materials.

9. The nicotine pellet composition according to claim 1, wherein the high polymer coating material comprises glycosyl based materials or high polymers, wherein the glycosyl based materials comprise any one or a combination of syrups, colored syrups, mucilages, talcum powders and cera chinensis; and the high polymers comprise any one or combination of hydroxypropyl methylcellulose, hydroxypropyl cellulose, hydroxyethyl cellulose, methyl cellulose, methacrylic acid copolymers, methacrylate copolymers, cellulose acetate phthalate, polyvinyl pyrrolidone, polyvinyl pyrrolidine and polyethylene glycol.

10. The nicotine pellet composition according to claim 1, wherein the lubricant comprise hydrophobic lubricants and hydrophilous lubricants, wherein the hydrophobic lubricants comprise any one or a combination of stearic acid, magnesium stearate, calcium stearate, glyceryl behenate, micronized silica, talc, vegetable oil, polyethylene glycol and sodium lauryl sulfate and the hydrophilous lubricants comprise sodium stearyl fumarate.

11. The nicotine pellet composition according to claim 1, wherein the flavoring comprises any one or a combination of bergamot flavor, eucalyptus flavor, citrus flavor, lemon flavor, peppermint flavor, mint flavor, menthol, licorice flavor, wintergreen flavor, tobacco flavor, coffee flavor, vanilla flavor, lime flavor, apple flavor, peach flavor, mango flavor, cherry flavor, blueberry flavor, strawberry flavor, cola flavor, cinnamon flavor and watermelon flavor.

12. The nicotine pellet composition according to claim 1, wherein the solvent comprises water and/or organic solvents, wherein the organic solvents comprise any one or a combination of aromatic hydrocarbons, aliphatic hydrocarbons, alicyclic hydrocarbons, halogenated hydrocarbons, alcohols, ethers, esters, ketones, diol derivatives, others such as acetonitrile, pyridine and phenol.

13. A nicotine pouch, comprising a non-woven pouch and the nicotine pellet composition as set forth in any of claims 1-12 in the non-woven pouch.

14. A preparation method of the nicotine pouch, for preparing the nicotine pouch, comprising:
S1: weighing and proportioning: weighing materials; proportioning the high polymer coating material according to a formulation;
S2: drug carrying and coating: after proportioning of the high polymer coating material, placing into a material silo having a sprayer, placing weighed solid materials sequentially into another material silo having a fluidized bed or a centrifugal coating machine, starting coating using the fluidized bed or the centrifugal coating machine, coating the solid materials evenly over the blank pellet core to form the single-layered or multi-layered coating and obtaining nicotine pellets;
S3: drying: placing the nicotine pellets into a drying box or the fluidized bed for drying; and
S4: packaging: transferring the nicotine pellets after drying to a powder silo of a granule packaging machine, placing rolls of non-woven cloths into a film silo of the granule packaging machine, starting the granule packaging machine for packaging, so to enclose the nicotine pellets into non-woven parcels and obtaining the nicotine pouch.
